# NEUE EUROPÄISCHE PATENTSCHRIFT

(11) **EP 0 478 932 B2**
(45) Veröffentlichungstag und Bekanntmachung des Hinweises auf die Entscheidung über den Einspruch: **02.05.1997**
(45) Hinweis auf die Patenterteilung: 15.12.1993
(21) Anmeldenummer: 91113530.9
(22) Anmeldetag: 13.08.1991
(51) Int. Cl.: A61K 6/04, C22C 5/02

(54) **Hochgoldhaltige gelbe Dentallegierung**
Yellow dental alloy with a high gold content
Alliage jaune dentaire à haute teneur en or

(30) Priorität: 03.10.1990 DE 4031169
(43) Veröffentlichungstag der Anmeldung: 08.04.1992
(73) Patentinhaber: Degussa Aktiengesellschaft, 60311 Frankfurt (DE)
(72) Erfinder: Schöck, Gernot, Dipl.-Ing., W-6454 Bruchköbel (DE); Kempf, Bernd, Dr. Dipl.-Ing., W-6364 Freigericht (DE); Groll, Werner, Dr. Dipl.-Ing., W-8755 Alzenau (DE)

(56) Entgegenhaltungen:
- CH-A- 636 905
- DE-B- 2 302 837
- DE-B- 2 424 575
- DE-C- 3 132 143
- JP-A-78 117 626
- US-A- 2 143 217
- R.M. German "Precious-metal dental casting alloys", in International Metal Reviews, 1982, vol. 27, n 5, S. 260-288
- "Das dental Vedemekum", 4. Auflage, Deutscher Ärzte-Verlag GmbH, Köln, 1989/1993, S. 820-835, 864, 865 und 873
- J.P. Nielsen, J.J. Tuccillo, J. Dent. Res., 1966, 45, S. 964-969
- Zeitschrift "Die Quintessenz der Zahntechnik, 12/1987, S. 1400-1401
- Thermodynamische Modellierung der Ausscheidungshärtung in Edelmetall-Legierungssystemen, Zeitschrift für Metallkunde, 86, 1985, S. 603-607

## Beschreibung

Die Erfindung betrifft eine hochgoldhaltige, gelbe Dentallegierung für mit Keramik verblendete und unverblendete Prothesenteile.

Edelmetallegierungen finden bei der Herstellung von metallischem, festsitzendem Zahnersatz eine breite Anwendung. Gründe dafür sind ihre günstigen Eigenschaften, wie hervorragende Korrosionsbeständigkeit, Biokompatibilität und gute Verarbeitbarkeit.

Goldgußlegierungen ohne keramische Verblendung werden daher schon seit mehreren Jahrzehnten in der Zahntechnik eingesetzt. Hochgoldhaltige Gußlegierungen enthalten neben Gold weitere Legierungselemente, um entsprechende Härten und mechanische Festigkeiten zu erreichen. Nach DIN 13906 teilt man die Gußlegierungen in die Typen 1 bis 4 ein, wobei der Typ 1 die geringsten Werte hinsichtlich Festigkeit und Härte besitzt und der Typ 4 die höchsten Werte. Legierungen mit hoher Festigkeit und Härte besitzen aufgrund der hohen mechanischen Stabilität den breitesten Indikationsbereich.

Bei den konventionellen Gußlegierungen wird die hohe Härte und Festigkeit der Typ 4-Legierungen im allgemeinen durch die Silber-Kupfer Mischungslücke erreicht.

Der Kupferzusatz in Goldlegierungen erhöht jedoch die Neigung zu Verfärbungserscheinungen im Mund. Man hat daher nach Wegen gesucht, auf das Kupferzu verzichten. In der DE-PS 21 39 331 sind entsprechende Legierungen beschrieben, die kein Kupfer enthalten, und bei denen die Härte in weiten Bereichen über die Höhe des Palladiumgehaltes einstellbar ist, wenn als weitere Legierungselemente Indium, Zinn, Zink und Platin zugegen sind. Um Legierungen vom Typ 4, d.h. mit einer Härte von mindestens 220 HV im ausgehärteten Zustand herzustellen, sind allerdings Palladiumgehalte von mindestens 6 % und Platingehalte von mindestens 2,4 % notwendig. Bei diesen Legierungen werden keine Verfärbungen im Munde beobachtet. Nachteilig ist aber, daß diese Legierungen durch das Fehlen des Kupfers und den relativ hohen Anteil des Weißmachers Palladium bzw. Platin den ansprechenden warmen Goldton verloren haben und nur noch hellgelb sind.

Aus ästhetischen Gründen werden speziell im Frontzahnbereich keramisch verblendete Konstruktionen verwendet, die beim heutigen Stand der Technik dem natürlichen Zahn so gut nachempfunden sind, daß nahezu keine Unterschiede mehr feststellbar sind.

Die keramische Verblendung erfordert spezielle Eigenschaften der Legierungen, die an die zur Verfügung stehenden keramischen Massen angepaßt sein müssen. So soll der thermische Ausdehnungskoeffizient der Legierung geringfügig kleiner sein, als der der Keramik, so daß die Keramik nach dem Abkühlungsprozeß unter Druckspannungen steht, da sie Druckspannungen in weit besserem Maße erträgt als Zugspannungen.

Auch muß die Legierung bei der für die bisher verfügbaren keramischen Massen notwendigen Aufbrenntemperatur von ca. 980° C noch die notwendige mechanische Stabilität besitzen, so daß es nicht zu Verbiegungen und damit zu einer Verschlechterung der Paßgenauigkeit der Prothesenteile kommt.

Diese Anforderungen führten in den letzten Jahrzehnten, seitdem brauchbare keramische Massen zur Verblendung zur Verfügung standen, zu einer großen Zahl von Legierungsentwicklungen. Es bildete sich damit eine eigene Legierungsklasse, die der Aufbrennlegierungen heraus, die sich von den vorher ausschließlich verwendeten Gußlegierungen deutlich abhoben.

Die Aufbrennlegierungen lassen sich in verschiedene Gruppen einteilen, und zwar in Gold-Basis-, Gold-Palladium-Basis- und Palladium-Silber-Legierungen. Allen Legierungen, selbst den hochgoldhaltigen, ist jedoch gemeinsam, daß sie den ansprechenden warmen Goldton der hochgoldhaltigen Gußlegierungen verloren haben, da größere Mengen an Palladium und Platin zulegiert werden müssen, um die oben zitierten Anforderungen bezüglich Hochtemperaturfestigkeit und angepaßtem thermischen Ausdehnungskoeffizienten zu erreichen. Entsprechende, hochgoldhaltige Aufbrennlegierungen sind z.B. in den DE-PSen 31 32 143 und 15 33 233 beschrieben.

Wegen der weniger ansprechenden Farbe und des hohen Schmelzbereichs werden die Aufbrennlegierungen im allgemeinen nicht als Gußlegierungen verwendet.

Für die ganze Palette zahntechnischer Arbeiten sind daher heute zwei verschiedene Legierungsklassen, nämlich die Aufbrennlegierungen und die Gußlegierungen notwendig.

Es wäre dahervon großem Vorteil, eine Dentallegierung zur Verfügung zu haban, die die Vorzüge der Gußlegierung hinsichtlich Farbe und Verarbeitung aufweisen würde und außerdem noch verblendfähig wäre. Da sich aus grundlegenden physikalischen Gründen der thermische Ausdehnungskoeffizient und das Schmelzintervall einer hochgoldhaltigen, geben Legierung nicht an die konventionellen Dentalkeramiken apassen läßt, ist eine derartige Legierung nur bei einer modifizierten Dentalkeramik möglich, die hinsichtlich thermischem Ausdehnungskoeffizient und Aufbrenntemperatur an die Legierung angenähert ist.

Seit jüngster Zeit stehen Dentalkeramiken zur Verfügung, die bei Temperaturen unterhalb 800° C aufgebrannt werden und thermische Ausdehnungskoeffizienten besitzen, die Im Bereich bis 17,5 · 10⁻⁶K⁻¹ liegen. Die bisher bekannten hochgoldhaltigen Gußlegierungen haben relativ hohe Anteile an Silber und Kupfer, um entsprechende Härten und Festigkeiten zu erhalten. Dadurch liegt der thermische Ausdehnungskoeffizient dieser Legierungen an der oberen Grenze der mit den neuen Dentalkeramiken maximal erreichbaren thermischen Ausdehnungskoeffizienten. Zur Erzielung hoher Verbundsicherheit zwischen Keramik und Metall muß der thermische Ausdehnungskoeffizient der Legierung jedoch niedriger liegen als der der Keramik.

Der relativ hohe Kupfer-Gehalt der bisherigen Gußlegierungen führt außerdem im Bereich der Verblendung zu einer dunklen, ästhetisch nicht akzeptablen Abweichung der Farbe der Dentalkeramik, da das dunkle Oxid durch die dünn auslaufende Keramik durchschimmert.

Kupferfreie Legierungen zeigen andererseits nur noch eine hellgelbe Farbe.

Es war daher Aufgabe der vorliegenden Erfindung, eine hochgoldhaltige gelbe Dentallegierungen für mit Keramik verblendete und unverblendete Prothesenteile zu entwickeln, deren thermische Ausdehnungskoeffizient unterhalb 17,5 · 10⁻⁶K⁻¹ liegt, keine Verfärbung der Keramikverblendungen bewirkt und die eine mit herkömmlichen hochgoldhaltigen Gußlegierungen vergleichbare gelbe Farbe aufweist.

Diese Aufgabe wird erfindungsgemäß dadurch gelöst, daß sie aus 70 bis 85 Gew.% Gold, 5 bis 13 Gew.% Silber, 2 bis 9 Gew.% Platin, 0 bis 4,5 Gew.% Palladium, 0,05 bis 1 Gew.% Iridium, Rhenium, Rhodium und/oder Ruthenium, 3 bis 8 Gew.% Kupfer, 0,1 bis 6 Gew.% Indium und Zink und 0 bis 4 Gew.% eines oder mehrerer der Metalle Gallium, Eisen und Wolfram besteht, wobei der Indiumgehalt mindestens 1 Gew.% betragen muß.

Vorzugsweise verwendet man Legierungen, die 7 bis 11 Gew.% Silber, 4 bis 7,5 Gew.% Platin, 0 bis 4,5 Gew.% Palladium, 0,05 bis 1 Gew.% Iridium, Rhenium, Rhodium und/oder Ruthenium, 3 bis 6 Gew.% Kupfer, 2 bis 4,5 Gew.% Indium und Zink, 0 bis 4 Gew.% Gallium, Eisen und/oder Wolfram, Rest Gold, enthalten, wobei der Indiumgehalt mindestens 1 Gew.% betragen muß.

Besonders bewährt haben sich Legierungen, die 0,2 bis 2 Gew.% Wolfram enthalten.

Es hat sich überraschenderweise gezeigt, daß bei einer Reduzierung des Kupfer- und Silber-Gehaltes im Vergleich zu bekannten Legierungen trotzdem die gelbe Farbe erhalten bleibt, wenn der Gehalt an Platinmetallen entsprechend gering gehalten wird.

Durch die Kombination der härtenden Wirkung des Kupfers und gleichzeitiger Zugabe von Indium und Zink lassen sich auch bei niedrigen Gehalten an Platin und Palladium Härten erreichen, die laut DIN für Typ 3- bzw. Typ 4-Legierungen notwendig sind. Insbesondere bei Indium-Zink-Gehalten von in der Summe mindestens 3,5 % lassen sich Härtewerte erreichen, die für Typ-4-Legierungen ausreichend sind. Außerdem zeigen diese Legierungen nach dem Oxidbrand ein sehr angenehmes, helles, gelbbraunes Oxid, das keine Verfärbung der Keramik bewirkt. Auch im thermischen Ausdehnungskoeffizienten liegen diese Legierungen im Bereich unterhalb 17,5 · 10⁻⁶K⁻¹. Die zur Erzielung der Härte notwendigen Platin- und Palladiumgehalte liegen so niedrig, daß die gelbe Farbe der Legierung nicht beeinträchtigt wird. Höhere Palladiumgehalte als 4,5 % sind nicht zulässig, da sich bei Anwesenheit der zulässigen Indium- und Zinkgehalten bis 6 Gew.%, die für die Härte notwendig ist, eine zweite Phase ausbildet, die die Duktilität der Legierungen verschlechtern würde.

Die erfindungsgemäßen Legierungen müssen zinnfrei sein, da Zinn die Duktilität der Legierungen stark vermindert und die Haftung der Keramikschichten verschlechtert.

Der Zusatz von 0,2 bis 2 Gew.% Wolfram führt zu glänzenden Oberflächen nach dem Ausbetten der Gußstücke.

Über die Zugabe der Elemente Eisen und Gallium lässt sich die Farbe der beim Aufbrennen entstehenden Oxidschicht beeinflussen. Außerdem ist damit eine Feinabstimmung der mechanischen Eigenschaften möglich.

Die folgende Tabelle zeigt die Eigenschaften einiger erfindungemäßer Legierungen.

**Tabelle**

| Leg. Nr. | Gold [Gew.%] | Silber [Gew.%] | Platin [Gew.%] | Palladium [Gew.%] | Ir/Re/Rh [Gew.%] | Kupfer [Gew.%] | Indium [Gew.%] | Zink [Gew.%] | Ga/Fe/W [Gew.%] |
|---|---|---|---|---|---|---|---|---|---|
| 1 | 74,7 | 9,2 | 4,4 | 2 | 0,1 | 4,4 | 4 | 1,2 | - |
| 2 | 75,4 | 9,2 | 4,4 | 3 | 0,1 | 4,4 | 1,5 | 2 | - |
| 3 | 76,7 | 9,2 | 4,4 | 2 | 0,1 | 4,4 | 2 | 1,2 | - |
| 4 | 74,7 | 9,2 | 6,4 | 2 | 0,1 | 4,4 | 2 | 1,2 | - |
| 5 | 74,4 | 9,2 | 6,4 | 2 | 0,1 | 4,4 | 1,5 | 2 | - |
| 6 | 75,8 | 9,2 | 5,4 | 2 | 0,1 | 4,4 | 1,5 | 1,5 | - |
| 7 | 74,7 | 9,2 | 5,4 | 2 | 0,1 | 4,4 | 2 | 1,2 | 1 (W) |
| 8 | 73,9 | 9,2 | 5,4 | 2 | 0,1 | 4,4 | 1,5 | 1,5 | 2 (Fe) |

**Tabelle**

| Leg. Nr. | Schmelzintervall Tₛₒₗ - T_{liq} [°C] | 0,2 % Dehngrenze [MPa] | Bruchdehnung [%] | Härte HVS | | Ausdehnungskoeff. [10⁻⁶K^{-1]} |
|---|---|---|---|---|---|---|
| | | | | Guß | Brand | |
| 1 | 950 - 945 | - | - | 184 | 191 | - |
| 2 | 923 - 993 | - | - | 207 | 204 | 17,2 |
| 3 | 899 - 988 | 434 | 18,2 | 195 | 183 | 17,0 |
| 4 | 923 - 1010 | 501 | 11,3 | 200 | 205 | 16,9 |
| 5 | 906 - 998 | 536 | 8,6 | 205 | 225 | 17,0 |
| 6 | 906 - 993 | 446 | 17,0 | 198 | 187 | 17,3 |
| 7 | 903 - 984 | - | - | 221 | 204 | 16,9 |
| 8 | 896 - 987 | - | - | 206 | 199 | 17,1 |

## Patentansprüche

1. Hochgoldhaltige gelbe Dentallegierung für mit Keramik verblendete und unverblendete Prothesenteile,
**dadurch gekennzeichnet**,
daß sie aus 70 bis 85 Gew.% Gold, 5 bis 13 Gew.% Silber, 2 bis 9 Gew.% Platin, 0 bis 4,5 Gew.% Palladium, 0,05 bis 1 Gew.% Iridium, Rhenium, Rhodium und/oder Ruthenium, 3 bis 8 Gew.% Kupfer, 2 bis 6 Gew.% Indium und Zink und 0 bis 4 Gew.% eines oder mehrerer der Metalle Gallium, Eisen und Wolfram besteht, wobei der Indiumgehalt mindestens 1 Gew.% betragen und die legierung zinnfrei sein muß.

2. Dentallegierung nach Anspruch 1,
**dadurch gekennzeichnet**,
daß sie aus 7 bis 11 Gew.% Silber, 4 bis 7,5 Gew.% Platin, 0 bis 4,5 Gew.% Palladium, 0,05 bis 1 Gew.% Iridium, Rhenium, Rhodium und/oder Ruthenium, 3 bis 6 Gew.% Kupfer, 2 bis 4,5 Gew.% Indium und Zink, 0 bis 4 Gew.% Gallium, Eisen und/oder Wolfram, Rest Gold besteht, wobei der Indiumgehalt mindestens 1 Gew. % betragen muß.

3. Dentallegierung nach Anspruch 1 oder 2,
**dadurch gekennzeichnet**,
daß sie 0,2 bis 2 Gew.% Wolfram enthält.

## Claims

1. Yellow dental alloy of high gold content for ceramic-faced and unfaced dentures,
**characterised in that**
it consists of 70 to 85 wt-% gold, 5 to 13 wt-% silver, 2 to 9 wt-% platinum, 0 to 4.5 wt-% palladium, 0.05 to 1 wt-% iridium, rhenium, rhodium and/or ruthenium, 3 to 8 wt-% copper, 2 to 6 wt-% indium and zinc and 0 to 4 wt-% of one or more of the metals gallium, iron and tungsten, it being essential for the indium content to amount to at least 1 wt-% and for the alloy to be free of tin.

2. Dental alloy as claimed in Claim 1,
**characterised in that**
it consists of 7 to 11 wt-% silver, 4 to 7.5 wt-% platinum, 0 to 4.5 wt-% palladium, 0.05 to 1 wt-% iridium, rhenium, rhodium and/or ruthenium, 3 to 6 wt-% copper, 2 to 4.5 wt-% indium and zinc, 0 to 4 wt-% gallium, iron and/or tungsten, the remainder being gold, it being essential for the indium content to amount to at least 1 wt-%.

3. Dental alloy as claimed in Claim 1 or 2,
**characterised in that**
it contains 0.2 to 2 wt-% tungsten.

## Revendications

1. Alliage dentaire jaune à haute teneur en or pour des pièces de prothèse non plaquées ou plaquées de céramique,
caractérisé en ce qu'
il est constitué de 70 à 85 % en poids d'or, 5 à 13 % en poids d'argent, 2 à 9 % en poids de platine, 0 à 4,5 % en poids de palladium, 0,05 à 1 % en poids d'iridium, de rhénium, de rhodium et/ou de ruthénium, 3 à 8 % en poids de cuivre, 2 à 6 % en poids d'indium et de zinc et de 0 à 4 % en poids d'un ou plusieurs des métaux gallium, fer et tungstène, il faut que la teneur en indium soit au moins de 1 % en poids et l'alliage doit être dépourvu d'étain.

2. Alliage dentaire selon la revendication 1,
caractérisé en ce qu'
il contient 7 à 11 % en poids d'argent, 4 à 7,5 % en poids de platine, 0 à 4,5 % en poids de palladium, 0,05 à 1 % en poids d'iridium, de rhénium, de rhodium et/ou de ruthénium, 3 à 6 % en poids de cuivre, 2 à 4,5 % en poids d'indium et de zinc, 0 à 4 % en poids de gallium, de fer et/ou de tungstène, le reste étant de l'or, il faut que la teneur en indium soit au moins de 1 % en poids.

3. Alliage dentaire selon la revendication 1 ou 2,
caractérisé en ce qu'
il contient 0,2 à 2 % en poids de tungstène.
